(19) Europäisches Patentamt
European Patent Office
Office européen des brevets

(11) EP 0 737 667 B1

(12) **EUROPÄISCHE PATENTSCHRIFT**

(45) Veröffentlichungstag und Bekanntmachung des Hinweises auf die Patenterteilung:
**07.11.2001 Patentblatt 2001/45**

(51) Int Cl.[7]: **C07C 209/84**

(21) Anmeldenummer: **96104874.1**

(22) Anmeldetag: **27.03.1996**

(54) **Fraktionierung und Reinigung von aromatischen Polyamingemischen und deren Verwendung**

Fractionation and purification of aromatic polyamine mixtures and their use

Le fractionnement et la purification des mélanges des polyamines aromatiques et leur utilisation

(84) Benannte Vertragsstaaten:
**BE DE ES FR GB IT NL PT**

(30) Priorität: **07.04.1995 DE 19513269**

(43) Veröffentlichungstag der Anmeldung:
**16.10.1996 Patentblatt 1996/42**

(73) Patentinhaber: **BAYER AG**
**51368 Leverkusen (DE)**

(72) Erfinder:
- **Knöfel, Hartmut, Dr.**
  **51519 Odenthal (DE)**
- **Brockelt, Michael**
  **51379 Leverkusen (DE)**

(56) Entgegenhaltungen:
**EP-A- 0 009 094**
**DE-A- 2 811 885**
**US-A- 3 996 283**
**US-A- 4 087 459**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe, wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyanate bzw. Isocyanatgemische fallen in vielen Fällen aber nicht in der Form und Zusammensetzung an, wie sie auf der Isocyanatstufe bevorzugt weiterverwendet werden, sondern müssen zuvor durch teilweise aufwendige Aufarbeitungs- und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder gar nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann. Diese Bestandteile sind zwar die Grundlage für ebenfalls begehrte Isocyanate, doch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, und kann nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u. a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE - A 2238319 , DE - A 2528694).

Die Effekte sind infolge der in einem solchen Gemisch vorhandenen zahlreichen Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

US 4 087 459 beschreibt ein Verfahren zur Herstellung mehrkerniger aromatischer Polyamine durch Kondensation von Anilin mit Formaldehyd in Gegenwart von Wasser und sauren Katalysatoren, wobei der erhaltene wäßrige Mischung der Kondensationsprodukte mit einem hydrophoben Lösungsmittel in einer Extraktionsstufe extrahiert wird, um den Anteil der para-Isomere in der Endproduktmischung anzureichern (quantitative Fraktionierung).

Aufgabe war es daher, ein Verfahren zur Verfügung zu stellen, das es gestattet, Polyamingemische in reinerer, neuartiger Zusammensetzung, wirtschaftlich und produktschonend, gezielt herzustellen.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z. B. auf der Isocyanatstufe schwierig durchzuführende Isomerentrennungen auf der Aminstufe vorwegnehmen. Solche Gemische können auch völlig neuartige, weil nach dem Stand der Technik nicht

darstellbare Polyamingemische sein, die zu völlig neuartigen Isocyanaten führen.

Andererseits kann das erfindungsgemäße Verfahren dazu genutzt werden, aus beliebigen, d. h. auch aus wiedergewonnenen Polyamingemischen, die sich durch Verunreinigung oder durch nichtstatistische, d. h. selektive Verluste bei einzelnen Komponeten bei der Wiedergewinnung von den ursprünglich eingesetzten Polyaminen oder Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt unerwünschte Neben- und Zwischenprodukte, mitzufraktionieren und in einer Produktfraktion ab - und in einer anderen entsprechend anzureichern, gegebenenfalls in einer eigenen Fraktion auszuschleusen.

Es handelt sich bei der vorliegenden Erfindung um ein breit anwendbares Verfahren, mit welchem die Aufgabe der Fraktionierung und Reinigung von aromatischen Diund Polyamingemischen, insbesondere der Diphenylmethanreihe, gelöst werden kann.

Gegenstand der Erfindung ist ein Verfahren zur Fraktionierung und Reinigung von aromatischen Polyamingemischen, verwendbar für die Herstellung von Polysocyanaten insbesondere von Polyamingemischen der Diphenylmethanreihe, welches dadurch gekennzeichnet ist, daß man

a) das Ausgangspolyamingemisch (A) in einem zweiphasigen System, bestehend aus (i) einer hydrophoben Lösungsmittelphase (B), die im wesentlichen aus hydrophobem Lösungsmittel besteht, und (ii) einer wäßrigen Phase (C), bestehend im wesentlichen aus Wasser, einer starken Säure und zumindest teilweise in der Salzform vorliegendem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer, nach dem Gegenstromprinzip arbeitenden Extraktionsstufe (3) unter Durchmischung der Phasen verteilt, indem man das Ausgangspolyamingemisch über die organische Phase (B) in die Extraktionsstufe (3) einbringt, und die die Extraktionsstufe verlassende organische Phase (D), zumindest teilweise, in einer mehrstufigen Destillation (6.1), (6.2) in eine erste, in der Extraktionsstufe (3) wiederverwendete Fraktion (E), bestehend im wesentlichen aus hydrophobem Lösungsmittel, eine zweite Fraktion (F), bestehend im wesentlichen aus Hilfsamin und einem Destillationsrückstand (G), bestehend im wesentlichen aus einer ersten Polyaminfraktion auftrennt und die die Extraktionsstufe (3) verlassende wäßrige Phase (H),

b) zumindest teilweise über eine zwischengeschaltete Extraktionsstufe (4)

c) in eine Extraktionsstufe (5) leitet, in welcher nach dem Prinzip der Gegenstromextraktion eine Extraktion der wäßrigen Phase mit einer aus Lösungsmittel und Hilfsamin bestehenden Lösungsmittelphase (J) erfolgt, und die an Polyarylamin verarmte wäßrige Phase (K) resultiert, die man

d) zumindest teilweise über eine zwischengeschaltete Destillationsstufe (8) führt und anschließend mit dem verbliebenen Rest von (K) vereinigt und

e) als Mengenstrom (C) wiederverwendet, in dem man besagte wäßrige Phase

f) zumindest teilweise über eine vorgeschaltete Extraktionsstufe (2)

g) der Extraktionsstufe (3) zuführt, gegebenenfalls nach Zugabe von Hilfsamin und/oder Wasser, und man

h) die in der Extraktionsstufe anfallende organische Phase (L) zumindest teilweise destillativ (7.1) in ein aus Hilfsamin und Lösungsmittel bestehendes Destillat (M) und einen aus einer zweiten Polyaminfraktion bestehenden Destillationsrückstand (N) zerlegt, wonach das Destillat (M) mit wenigstens einem Teil des in der Destillationsstufe (6.2) gewonnenen Destillates (F) vereinigt und anschließend zur Extraktionsstufe (5) zurückgeführt und dort wieder verwendet wird.

Das erfindungsgemäße Verfahren wird bevorzugt so durchgeführt, daß

Die in der Extraktionsstufe (3) anfallende wäßrige Phase (H) zumindest teilweise in einer zwischengeschalteten, nach dem Gegenstromprinzip arbeitenden Extraktionsstufe (4) extrahiert unter Verwendung einer organischen Phase (O) als Extraktionsmittel, bestehend neben hydrophobem Lösungsmittel aus Hilfsamin und gegebenenfalls Polyamin, letzteres vorzugsweise mit einer Zusammensetzung des zweiten Teilproduktes (N) und vorzugsweise eingebracht als Teilmenge des Mengenstromes (L), die in Verfahrensstufe (4) resultierende organische Phase (P) dem Mengenstrom (B) und damit der Extraktionsstufe (3) und die resultierende wäßrige Phase (Q) der Extraktionsstufe (5)

zuführt.

Das erfindungsgemäße Verfahren wird besonders bevorzugt so durchgeführt, daß man die die Extraktionsstufe (5) verlassende wäßrige Phase (K) vor ihrer Wiederverwendung zumindest teilweise destillativ (8) von einem Teil (X) des in ihr enthaltenen Wassers befreit, dieses gegebenenfalls zum Waschen (6.0) des der destillativen Aufarbeitung (6.1), (6.2) zugeführten Teiles der die Extraktionsstufe (3) verlassenden organischen Phase (D) und/oder zum Waschen (7.0) des der destillativen Aufarbeitung (7.1) zugeführten Teiles der die Extraktionsstufe (5) verlassenden organischen Phase (L) zwecks Entfernens von Säurespuren verwendet, und das hierbei anfallende Wasser (Y) an geeigneter Stelle in die wäßrige Phase zurückführt.

Eine weiter verbesserte und daher bevorzugte Ausführungsform des erfindungsgemäßen Verfahrens wird dadurch erreicht, daß man eine Teilmenge (D") der die Extraktionsstufe (3) verlassenden organischen Phase (D) abtrennt und in einer vorgelagerten, vorzugsweise mehrstufigen, Extraktionsstufe (2) mit zumindest einer Teilmenge, vorzugsweise mit dem gesamten Mengenstrom (C) im Gegenstrom extrahiert, und den Teilmengenstrom (D") so bemißt, daß dabei ein möglichst weitgehender Übergang des in (D") enthaltenen Polyamins in die die Extraktionseinheit (2) verlassende wäßrige Phase stattfindet, besagte wäßrige Phase, gegebenenfalls nach Zugabe von Wasser aus Mengenstrom (Y) und/oder Hilfsamin und/oder weiterer wäßriger Phase aus Mengenstrom (C) der Extraktionsstufe (3) zuführt, und die in (2) anfallende organische Phase (R), bestehend im wesentlichen aus hydrophobem Lösungsmittel und gegebenenfalls Hilfsamin, ebenfalls der Extraktionsstufe (3) zuführt und als Bestandteil der organischen Phase (B) als Lösungsmittel für das Ausgangspoplyamin (A) verwendet.

Als Hilfsamin wird vorzugsweise Anilin eingesetzt und als Polyamingemisch der Diphenylmethanreihe bevorzugt ein Polyamingemisch, wie es bei der säurekatalysierten Anilin/Formaldehyd-Kondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung entsprechender kernhydrierter Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind

1. Polyamingemische der Diphenylmethanreihe, wie sie bei der Kondensation und säurekatalysierten Umlagerung von Anilin mit Formaldehyd entstehen,

2. Polyamingemische der Diphenylmethanreihe, wie sie bei der säurekatalysierten Kondensation von substituierten Anilinen mit Formaldehyd anfallen,

3. Polyamingemische der Diphenylmethanreihe, wie sie bei der Mischkondensation von substituierten Anilinen untereinander und/oder mit Anilin mit Formaldehyd anfallen,

4. Polyamingemische der Diphenylmethanreihe, wie sie bei der Kondensation, auch der Mischkondensation von substituierten Anilinen und/oder Anilin mit Aldehyden und/oder Ketonen anfallen,

5. Polyamingemische der Diphenylmethanreihe, wie sie bei der Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,

6. Polyamingemische der Diphenylmethanreihe, wie sie bei der Kondensation von Monoarylmonoaminen (z. B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und

7. Polyamingemische der Triphenylmethanreihe, wie sie z. B. bei der Nitrierung und anschließenden Reduktion von Triphenylmethan, insbesondere alkylsubstituierten Triphenylmethanen und seinen höherkernigen, insbesondere Benzylhomologen entstehen.

Bei den zum Einsatz gelangenden hydrophoben Lösungsmitteln handelt es sich um inerte Lösungsmittel des

Siedepunktbereiches von 30° - 280°C, vorzugsweise von 80° - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d. h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5, vorzugsweise unter 1,5 liegeden pKA-Wert. Beispiele hierfür sind Salzsäure, Bromwasserstoffsäure, Schwefelsäure, Trifluoressigsäure, Methansulfonsäure oder Phosphorsäure.

Bevorzugt eingesetzt werden Salzsäure und Methansulfonsäure. Die genannten Säuren können auch im Gemisch mit sauren oder neutralen Salzen derartiger Säuren, wie z.B. den entsprechenden Ammoniumsalzen oder auch den entsprechenden Alkalisalzen, eingesetzt werden. Die genannten Säuren werden nicht in freier Form eingesetzt, sondern liegen in dem erfindungsgemäßen Kreislaufsystem in Form der entsprechenden Ammoniumsalze der in den wäßrigen Kreislaufsystem befindlichen Basen vor. Das sind in der Regel Polyamingemische von der Art der Ausgangsgemische und/oder die verwendeten Hilfsamine.

Als Hilfsamin finden in der Regel Monoarylamine, wie z. B. Anilin und/oder am Kern und/oder am Stickstoff alkylsubstituierte Anilinderivate Verwendung. Bevorzugt werden primäre Aniline eingesetzt, besonders bevorzugt ist Anilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann zur Steigerung des Anreicherungs- bzw. korrespondierenden Abreicherungseffektes mit jeder der anfallenden Produktfraktionen wiederholt werden.

Das erfindungsgemäße Verfahren kann sowohl mit zwei (Abb. 1) als auch mit drei (Abb. 2 und Abb. 3) oder mit vier Extraktionsstufen (Abb. 4) durchgeführt werden.

Die in Abb. 1-4 dargestellten Fließdiagramme dienen der weiteren Erläuterung des erfindungsgemäßen Verfahrens. In diesen Abbildungen bedeuten:

- (1) einen Tank für das Ausgangsarylamingemisch
- (2) eine vorgeschaltete Extraktionsstufe
- (3) eine erste Extraktionsstufe
- (4) eine (zwischengeschaltete) zweite Extraktionsstufe
- (5) eine (letzte) dritte Extraktionsstufe
- (6) eine Aufarbeitungsstufe bestehend aus
    - (6.0) einer Waschstufe
    - (6.1) einer ersten Destillationsstufe einer Mehrstufendestillation
    - (6.2) einer letzten Destillationsstufe einer Mehrstufendestillation
- (7) eine weitere Aufarbeitungsstufe bestehend aus
    - (7.0) einer Waschstufe
    - (7.1) einer Destillationsstufe
- (8) einen Wasserverdampfer
- (9) einen Tank für ein Verfahrensprodukt
- (10) einen Tank für ein weiteres Verfahrensprodukt.

Die Bezugszeichen A-R, U, X und Y bezeichnen die Mengenströme, auf die nachstehend und in den Beispielen Bezug genommen wird.

Die vorgeschaltete Extraktionsstufe (2) wird vorzugsweise als mehrstufig wirkender Extraktor ausgeführt.

Bei der Extraktionsstufe (3) handelt es sich im einfachsten Fall um eine einstufig wirkende Mischer-Scheidereinheit, vorzugsweise kommen jedoch mehrstufig wirkende Extraktionseinheiten zum Einsatz.

Die gegebenenfalls zwischengeschaltete Extraktionsstufe (4) besteht im einfachsten Falle ebenfalls aus einer Mischer-Scheidereinheit, vorzugsweise kommen jedoch auch hier mehrstufig wirkende Extraktionseinheiten zum Einsatz.

Die letzte Extraktionsstufe (5) wird im allgemeinen als mehrstufig wirkender Extraktor ausgeführt.

Die Aufarbeitungsstufen (6) und (7) dienen der Abtrennung der Polyaminfraktionen, welche als Destillationsrückstände anfallen und als Verfahrensprodukte (G) und (N) in den Tanks (9) und (10) isoliert werden, und der Wiedergewinnung des eingesetzten hydrophoben Lösungsmittels und des verwendeten Hilfsamins als Destillate.

Es hat sich als zweckmäßig erwiesen, die den Destillationsstufen zugeführten organischen Phasen (D) bzw. (D') und (L) bzw. (L') vor ihrer destillativen Behandlung in vorgelagerten Waschstufen (6.0) und (7.0) durch Extraktion mit Wasser von anhaftenden Säurespuren zu befreien.

Die eigentliche Aufarbeitungsstufe (6) besteht im allgemeinen aus einer wenigstens zweistufigen Mehrstufendestillation, deren erste Stufe (6.1) ein gegenüber dem Zulaufprodukt (D) bzw. (D') von Polyarylamin befreites und an Hilfsamin verarmtes hydrophobes Lösungsmittel als Destillat (E) liefert, und deren letzte Stufe (6.2) ein gegenüber (D) bzw. (D') von Polyarylamin befreites und an hydrophobem Lösungsmittel verarmtes Hilfsamin als Destillat (F) liefert.

Die vollständige destillative Auftrennung von hydrophoben Lösungsmittel und Hilfsamin ist bei der Durchführung des erfindungsgemäßen Verfahrens nicht erforderlich.

Zusätzlich fällt in der letzten Destillationsstufe (6.2) die im Mengenstrom (D) bzw. (D') enthaltene erste Polyaminfraktion des Ausgangsamingemisches (A) als Destillationssumpf (G) an.

Die Aufarbeitungsstufe (7) besteht im einfachsten Fall aus einer Destillationskolonne (7.1), die so ausgelegt ist, daß eine weitgehende Abtrennung von hydrophobem Lösungsmittel und Hilfsamin gemeinsam als Destillat (M) von der im Zulauf zu (6) enthaltenen Polyaminfraktion (N) destillativ erfolgt.

Vorzugsweise wird jedoch auch die Aufarbeitungsstufe (7) des erfindungsgemäßen Verfahrens wegen der verbesserten Energienutzung als mehrstufige Destillation ausgelegt.

Bei der Destillationsstufe (8) handelt es sich um eine Vorrichtung, mit welcher der wäßrigen Phase des Systems oder einem Teilstrom der wäßrigen Phase destillativ Wasser entzogen werden kann.

Ein solcher Schritt ist für die Durchführung des erfindungsgemäßen Verfahrens grundsätzlich nicht erforderlich, wegen der sich ergebenden Vorteile sind die Ausführungsformen unter Einschluß einer Wasserdestillation (8) jedoch bevorzugt.

Bei der wäßrigen, die Säure enthaltenden Phase liegt praktisch ein geschlossenes Kreislaufsystem vor, so daß die Stufe (8) grundsätzlich an einer beliebigen Stelle dieses Kreislaufsystems eingeschoben werden kann. Die Position von Stufe (8) in Anschluß an die Extraktionsstufe (5) und vor Eintritt in die Extraktionsstufe (2) bzw. (3) ist die vorteilhafteste und daher die bevorzugte Ausführungsform.

Die entnommene Wassermenge (X) wird gegebenenfalls nach ihrer Aufteilung in Teilströme und deren unterschiedlicher Verwendung, in Form des Mengenstromes (Y) insgesamt oder in Teilströmen dem System an geeigneter Stelle wieder zugeführt, so daß ein erweitertes und gegebenenfalls verzweigtes in sich geschlossenes wäßriges Kreislaufsystem entsteht.

Dieses schließt auch die Waschstufen (6.0) und/oder (7.0) mit ein. Letztere sind ein- oder mehrstufig wirkende nach dem Gegenstromprinzip arbeitende Extraktionsstufen. In Waschstufe (6.0) wird die organische Phase (D) bzw. (D') mit einem Teilstrom von (X) von anhaftenden Säurespuren befreit, in der Waschstufe (7.0) wird die organische Phase (L) bzw. (L') unter Verwendung eines anderen Wasserteilstromes von (X) von anhaftenden Säurespuren befreit.

Das mit hydrophobem Lösungsmittel und Hilfsamin contaminierte Destillat (X) ist für die Waschstufen (6.0 ) und (7.0) bestens geeignet. Die resultierenden Waschwässer weisen in der Regel eine sehr viel geringere Säurekonzentration auf als der eigentliche Säurekreislauf, so daß diese problemlos in Form des Mengenstromes (Y) oder seiner Teilströme recyclisiert werden können, gegebenenfalls kann eine Destillatteilmenge von (X) an den Waschstufen vorbei nach (Y) geführt und zur Aussteuerung eines unterschiedlichen Wassergehaltes der wäßrigen Phase in den einzelnen Extraktionsstufen verwendet werden.

Die praktisch quantitative Kreislaufführung der verwendeten Säure ermöglicht den Einsatz kostspieliger Säuren wie z. B. Methansulfonsäure, die wiederum wegen ihrer geringeren Korrosionsneigung die Verwendung kostengünstiger Werkstoffe in den Apparaten des erfindungsgemäßen Verfahrens gestattet.

Es hat sich als zweckmäßig erwiesen, den Säuregehalt der wäßrigen Phase, unabhängig von dem sich in der wäßrigen Phase eines zweiphasigen Systems einstellenden unterschiedlichen Amingehalt, über eine sogenannte "Molarität" zu definieren.

Die "Molarität" wird festgelegt als theoretische Konzentration von zu 100 % protoniertem Amin (d. h. gleiche Anzahl von Säure- und Aminäquivalenten) in einem rechnerisch um den Anteil an nicht protonierten Amin vermindertem

Volumen an wäßriger Phase gemäß der Formel

$$\text{"Molarität"} = \frac{\text{Mol 100 \% protoniertes Amin}}{\text{Vol. wäßrige Phase - Vol. unprotoniertes Amin}}$$

Die so definierte Molarität kann Werte bis 6 annehmen und wird je nach der der jeweiligen Ausführungsform zugrunde liegenden - hier produktbezogenen - Trennaufgabe in diesem Bereich gezielt variiert.

Auch innerhalb einer Ausführungsform des erfindungsgemäßen Verfahrens ist es gegebenenfalls vorteilhaft, die einzelnen von der wäßrigen Phase durchlaufenen Verfahrensstufen, insbesondere die Extraktionsstufen (2) bis (5) mit unterschiedlicher Molarität in der wäßrigen Phase zu betreiben, indem der wäßrigen Phase zwischen den einzelnen Stufen Wasser entzogen oder zugeführt wird.

Nach oben wird dieser Arbeitsbereich technisch begrenzt einerseits durch zunehmende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Nach unten wird dieser Bereich wirtschaftlich begrenzt durch den abnehmenden Säuregehalt und damit die quantitative Abnahme der Trennleistung, d.h. bei hervorragender qualitativer Trennleistung und technisch problemlos, ist mit sinkender Molarität ein zunehmend größeres Volumen an wäßriger Phase erforderlich für die Trennung einer gegebenen Aminmenge.

Der Protonierungsgrad gibt das Verhältnis von Säureäquivalenten zu Aminäquivalenten wieder.

Gemäß einer Variante des erfindungsgemäßen Verfahrens erfolgt die Einspeisung des Ausgangspolyamingemisches (A) aus dem Vorratsbehälter (1) durch Vermischen mit dem Strom (B), gebildet aus dem Mengenstrom (E) der ersten Destillationsstufe (6.1), bestehend aus hydrophobem Lösungsmittel und gegebenenfalls Hilfsamin, und gegebenenfalls einem Teilstrom des Destillates (F) der zweiten Destillationsstufe (6.2).

Im allgemeinen liegt der Gehalt an Arylamin, bestehend aus Ausgangspolyamin und Hilfsamin in Mengenstrom (B) nach Zugabe von (A) bei 10 - 80 Gew.-% vorzugsweise bei 15 - 60 Gew.%.

Der Gehalt an Ausgangspolyamin in Mengenstrom (B) nach Zugabe von (A) liegt im allgemeinen bei 5 - 60 Gew.-%, vorzugsweise bei 10 - 40 Gew.%. Im Extraktor (3) wird der mit (A) beaufschlagte Mengenstrom (B) der wäßrigen Phase (C) entgegengeführt.

Im allgemeinen besteht der Mengenstrom (C) aus Wasser, einer starken Protonsäure, Hilfsamin und gegebenenfalls Polyamin.

Die Säure liegt in Form ihrer in Wasser gelösten Salze mit Hilfsamin und gegebenenfalls mit Polyamin vor. Die Aminogruppen von Hilfsamin und gegebenenfalls Polyamin liegen in (C) stets im stöchiometrischen Überschuß vor, bezogen auf die Säure.

Der Protonierungsgrad beträgt in (C) im allgemeinen 10 - 90 %, für das vorzugsweise als Hilfsamin verwendete Anilin liegt er vorzugsweise bei 25 -70 %.

Die für die jeweilige Ausführungsform des erfindungsgemäßen Verfahrens wohldefinierte und in engen Grenzen gemessene und geregelte Molarität des Mengenstrom (C) wird je nach der der jeweiligen Ausführungsform zugrunde liegenden - hier produktbezogenen - Trennaufgabe in einem weiten Bereich gezielt varriiert.

Im allgemeinen hat die der ersten Extraktionsstufe des erfindungsgemäßen Verfahrens zugeführte wäßrige Phase (C) eine Molarität zwischen 0,5 und 4,5.

In der vorzugsweise mehrstufig betriebenen Extraktionsstufe (3) wird der mit (A) beaufschlagte Mengenstrom (B) der wäßrigen Phase (C) entgegengeführt.

Bei diesem Vorgang findet ein Übergang von Polyamin aus der organischen Phase (B) in die wäßrige Phase statt, in der Regel zumindest teilweise im Austausch von Hilfsamin in entgegengesetzter Richtung.

In der die Extraktionsstufe (3) verlassenden wäßrigen Phase (H) liegt die Säure als wäßrige Lösung ihrer Ammoniumsalze mit Polyamin und gegebenenfalls Hilfsamin vor, die in der Regel freies, d.h. nicht salzartig gebundenes Polyamin und gegebenenfalls freies, d.h. nicht salzartig gebundenes Hilfsamin gelöst enthält.

Das zusammen mit der organischen Phase (B) in den Extraktor (3) eingebrachte Ausgangspolyamin (A) verteilt sich auf die den Extraktor verlassende wäßrige Phase (H) und die den Extraktor (3) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponente in der Regel die orthoisomere(n) Form(en) in der die Trennstufe (3) verlassenden organischen Phase (D) relativ angereichert ist (sind); beispielsweise 2.4'-Diamino-diphenylmethan relativ zu 4,4'-Diamino-diphenylmethan. Umgekehrt ist die resultierende wäßrige Phase (H) relativ verarmt an dem 2,4'-Isomeren, während das 4,4'-Isomere relativ angereichert ist.

Sind mehrere "ortho-Isomere" im Ausgangspolyamin vorhanden, z. B. 2,2'- und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem "noch ortho-ärmeren" 4,4'-Isomeren relativ angereichert ist.

Der zuerst bei den Anilinformaldehydkondensationsprodukten der Diamino-diphenylmethanreihe gefundene An- und Abreicherungseffekt wurde rein empirischdeskriptiv mit dem Kriterium der ortho- und para-Substitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Substitutionsgrad" wird dabei das Verhältnis der orthoständigen Aminogruppen-Methylengruppenrelationen, zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad - auch für die gut charakerisierten und analytisch erfaßbaren isomeren Dreikernverbindungen aus der Anilin-Formaldehydkondensation gefunden.

Analoges gilt für die Trennung der Isomeren von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung, Aminogruppen auf, die praktisch nur orthoständig und/oder paraständig zu Methylengruppen sind.

Dabei werden innerhalb einer Gruppe isomerer Verbindungen in der Regel diejenigen mit dem höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und para-ständigen Aminogruppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

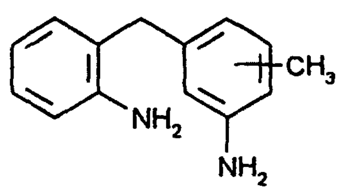

und

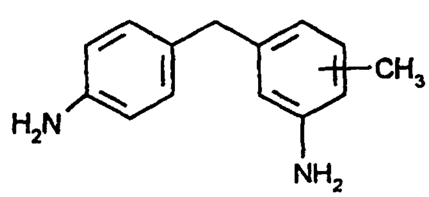

Bei der Fraktionierung solcher Gemische mit Hilfe des erfindungsgemäßen Verfahrens werden die 3,2'-Amino-Isomeren in der organischen Phase (D) gegenüber den 3,4'-Amino-Isomeren angereichert.

Das Kriterium "orthoreich" und "orthoarm" oder der ortho-Substitutionsgrad erfaßt in diesen Polyamingemischen nicht mehr alle Isomeren und ist daher sinngemäß anzuwenden, indem anstelle der Begriffe "orthoständig" und

"paraständig" eine Einteilung der Isomeren vorgenommen wird in solche mit kleinerem (= ortho-) und solche mit größerem (= para-) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam franktionieren lassen, stellen die Polyamine des Triphenylmethans und seiner höherkernigen Homologen, vorzugsweise Benzylhomologen dar, wie sie z. B. durch Nitrierung und anschließende Reduktion der entsprechenden Kohlenwasserstoffgemische hergestellt werden.

Bei der Fraktionierung technischer Polyamingemische der zuletzt genannten Substanzklassen

I. Mischkondensationsprodukte von Mono- und Diaminoarylverbindungen mit Formaldehyd bzw. allgemein Carbonylverbindungen,

II. Polyamingemische aus Verfahren durch Nitrierung und anschließende Reduktion von Diphenylmethan und vorzugsweise substituierten insbesondere alkylsubstituierten Diphenylmethanen und den jeweiligen Homologen und

III. Polyamingemische aus Verfahren durch Nitrierung und anschließende Reduktion von Triphenylmethan und vorzugsweise substituierten insbesondere alkylsubstituierten Triphenylmethanen und den jeweiligen höherkernigen Benzylhomologen

wurde zusätzlich zur reinen Isomerentrennung eine weitere überraschende Selektivität gefunden.

Polyamingemische der genannten Substanzklasse I bis III enthalten oder können enthalten Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit der in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0, für Phenylendiamin und seine Kondensationsprodukte stets 2,0. Für reine Mischkondensate ergeben sich für die Diphenylmethanisomeren der Wert 1,5 und für die höherkernigen Homologen Werte zwischen $> 1,0$ und $< 2,0$. Bei statistischer Verwendung des Begriffes Aminosubstitutionsgrad zur Charakterisierung von technischen Polyamingemischen ergeben sich ebenfalls Werte zwischen 1,0 und 2,0.

Bei der Fraktionierung von Polyamingemischen mit Komponenten mit einem Aminosubstitutionsgrad $> 1,0$ wurde nun gefunden, daß die Komponenten mit einem höheren Aminosubstitutionsgrad in der im eigentlichen Trennschritt resultierenden wäßrigen Phase relativ angereichert werden, und zwar um so stärker je größer der Aminosubstitutionsgrad ist.

Unabhängig davon ist auch hier die Trennung nach ortho-Substitutionsgrad wirksam.

Somit eröffnet das erfindungsgemäße Verfahren auch für diese Substanzklasse die Möglichkeit die Herstellungsform der Rohstoffe (Aminstufe) und die Verwendungsform der Endprodukte (Isocyanatstufe) zu entkoppeln, so daß eine getrennte Optimierung beider Stufen erleichert wird bis hin zur Gewinnung völlig neuer Isocyanatgemische.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wir der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches auszudrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (3).

Eine hohe Molarität der wäßrigen Phase in (3) innerhalb des angegebenen Molaritätsbereiches führt zu einer relativen Abreicherung höherkerniger Komponenten in der organischen Phase (D) und dementsprechend zu einer relativen Anreicherung in der wäßrigen Phase (H).

Eine niedrige Molarität der wäßrigen Phase in (3) innerhalb des angegebenen Molaritätsbereiches führt zu einer relativen Anreicherung höherkerniger Komponenten in der organischen Phase (D).

Der überraschende Befund kann dahingehend erweitert und präzisiert werden, das die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d. h. eine noch stärkere relative An- und Abreicherung, gefunden, desgleichen von Fünfkernkomponenten gegenüber Vierkernkomponenten usw.

Daraus und aus der gleichzeitg und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit geänderten Verfahrensparametern, zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanato-diphenylmethanreihe und ganz besonders für Polyamin- und Polyisocyanatgemische mit einem exterem hohen Anteil an höherkernigen Komponenten.

Die An- bzw. Abreicherung wird in der Regel effektiver mit steigendem Protonierungsgrad in der wäßrigen Phase der Trennstufe.

Darüber hinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege angereichert werden oder als gezielt hergestellte Polyamingemische, wie z. B. Polyaminobenzophenone oder Aminobenzylarylamingemische ihrerseits fraktioniert werden.

Die die Extraktionsstufe (3) verlassende, organische Phase (D) enthält unter anderem noch geringe Mengen an Säure, im allgemeinen und in Abhängigkeit von den Verfahrensparametern in der Extraktionsstufe (3) zwischen 0,01 und 0,5 Gew.-%, die vorteilhaft vor der destillativen Aufarbeitung des Mengenstromes (D) entfernt werden.

Im einfachsten Falle geschieht dies durch Neutralisation mit überschüssigen, verdünnten wäßrigen Basen, beispielsweise verdünnter Natronlauge. Bevorzugt wird jedoch das Herauswaschen der Säure bzw. ihrer Aminsalze aus der organischen Phase mit Wasser, so daß gegebenenfalls nur noch verbleibende Spuren durch Kontakt mit verdünnter Natronlauge oder mit Hilfe eines Ionentauschers entfernt werden.

Das eingesetzte Waschwasser wird unter Einschaltung eines Wasserverdampfers dem wäßrigen Säurekreislauf entzogen und diesem nach Durchlaufen der Waschstufe(n) mitsamt der Säure an verfahrensmäßig geeigneter Stelle wieder zugesetzt.

Die organische Phase (D) bzw. (D') wird, gegebenenfalls nach Durchlaufen der Säurewaschstufe (6.0), in die wenigstens zweistufige Destillationsstufe (6.1) (6.2) überführt.

In der ersten Destillationsstufe (6.1) wird ein Destillat (E) abgetrennt, welches die Hauptmenge, vorzugsweise nahezu die Gesamtmenge des in (D) bzw. (D') enthaltenen, hydrophoben Lösungsmittels neben einem Teil des in ihr enthaltenen Hilfsamins umfaßt. Im allgemeinen enthält das Destillat (E) < 50 % Hilfsamin, vorzugsweise 15 - 30 %.

In der letzten Destillationsstufe (6.2) wird das verbleibende Hilfsamin, gegebenenfalls neben der Restmenge des hydrophoben Lösungsmittels, als Destillat (F) von dem als Destillationssumpf anfallenden und im Verfahrensprodukttank (9) gesammelten ersten Teilprodukt (G) abgetrennt. Im allgemeinen enthält (F) < 50 % hydrophobes Lösungsmittel, vorzugsweise 15 - 30 %.

Das entsprechende zweite Verfahrensteilprodukt befindet sich in der die Extraktionsstufe (3) verlassenden wäßrigen Phase (H).

In einer mehrstufig wirkenden, vorzugsweise bei 80 - 110°C betriebenen Extraktionsstufe (5) wird aus der wäßrigen Phase (H), gegebenenfalls nach Zugabe von Wasser zur Absenkung der Molarität und gegebenenfalls nach Zugabe von Hilfsamin zur Absenkung des Protonierungsgrades, das zweite Teilprodukt im Austausch gegen Hilfsamin extrahiert und dabei in die organische Phase (L) überführt.

Die Molarität der in (5) eingesetzten wäßrigen Phase beträgt vorzugsweise < 2,5.

Der Protonierungsgrad der in (5) eingesetzten wäßrigen Phase ist vorzugsweise < 60 %.

Als Extraktionsmittel (J) dient ein Gemisch aus hydrophobem Lösungsmittel und Hilfsamin, welches im wesentlichen aus der Destillatfraktion (M) der Destillationsstufe (7.1) und einer Teilmenge, der Destillatfraktion (F) der

Destillationsstufe (6.2) zusammengesetzt wird.

Das Gewichtsverhältnis von Hilfsamin zu Lösungsmittel liegt in (J) im allgemeinen zwischen 0,5 : 1 und 3 : 1, vorzugsweise zwischen 1 : 1 und 2 : 1.

Das Gewichtsverhältnis von Extraktionsmittel (J) zu wäßriger Phase liegt im allgemeinen zwischen 0,3 : 1 und 3 : 1, vorzugsweise zwischen von 0.7 : 1 und 2 : 1.

Die in (5) resultierende, organische Phase (L) bzw. (L') wird, gegebenenfalls nach Durchlaufen der Waschstufe (7.0) und/oder gegebenenfalls nach Entfernen von Säurespuren mit verdünnter Natronlauge, der Destillationsstufe (7.1) zugeführt.

In der Destillationsstufe (7.1) erfolgt die destillative Abtrennung des Destillationsrückstandes (N), der als zweites Teilprodukt in dem Verfahrensprodukttank (10) gesammelt wird.

Die Destillationsstufe (7.1) kann beispielsweise aus einem einstufigen Verdampfer bestehen, der neben dem Destillationsrückstand (N) ein Destillat (M) liefert.

Das Destillat (M) enthält neben Hilfsamin das gesamte hydrophobe Lösungsmittel aus (L) bzw. (L') und wird nach Zusatz zumindest einer Teilmenge von (F) als Extraktionsmittel (J) verwendet. Für den allgemeinen Fall, daß (F) neben Hilfsamin auch hydrophobes Lösungsmittel enthält, erfolgt ein entsprechender Bilanzausgleich an hydrophobem Lösungsmittel hin zu (E), d. h. zu dem organischen Kreislauf zur Gewinnung des ersten Teilproduktes (nicht gezeichnet).

Die in (5) resultierende wäßrige Phase (K) wird zur Extraktionsstufe (3) zurückgeführt, vorzugsweise zumindest teilweise über eine Destillationsstufe (8), in welcher der wäßrigen Phase die Wassermenge (X) entzogen wird.

Der Weg über (8) ist dann zwingend erforderlich, wenn die Extraktionsstufe (3) bei einer höheren Molarität der wäßrigen Phase betrieben wird als die Extraktionsstufe (5). In diesem Falle erfolgt die zumindest anteilmäßige Rückspeisung von Wasser (Y) in den wäßrigen Säurekreislauf nach Verlassen der Stufe (3) und vor Eintritt in die Stufe (5).

Grundsätzlich kann die in Verfahrensstufe (3) resultierende wäßrige Phase (H) direkt oder gegebenenfalls über ein Zwischenstufe (4) der Extraktionsstufe (5) zugeführt werden. Da aber der obere, für bestimmte Trennaufgaben durchaus bevorzugte Molaritätsbereich der Extraktionsstufe (3) oberhalb des bevorzugten Molaritätsbereichs der Extraktionsstufe (5) liegt, ist es eine bevorzugte Ausführungsform des erfindungsgemäßen Verfahrens, die Molaritäten in den verschiedenen Extraktionsstufen dadurch zu entkoppeln, daß dem in sich geschlossenen System der wäßrigen Phase gegebenenfalls an geeigneter Stelle destillativ Wasser entzogen und an anderer geeigneter Stelle wieder zugesetzt wird.

Mit Hilfe einer Wasserdestillationsstufe (8) wird der wäßrigen, die Säure enthaltende Phase, oder einem Teilstrom der wäßrigen Phase vorzugsweise nach Verlassen der Extraktionsstufe (5) und vor der Wiederverwendung am Prozeßbeginn Wasser entzogen, welches vor Eintritt der wäßrigen Phase in die Extraktionsstufe (5) insgesamt (Y) oder in Teilmengen an einer oder mehreren Stellen wieder zugesetzt wird.

Mit dieser ersten Variante des erfindungsgemäßen Verfahrens lassen sich beträchtliche Trennleistungen bei der Fraktionierung von Polyamingemischen erzielen und zahlreiche Trennprobleme zufriedenstellend lösen.

Insbesondere in der ersten Polyaminfraktion (G) kann die relative Anreicherung der in dieser Fraktion bevorzugt enthaltenen Komponeten gezielt variiert und maximiert werden.

Der in der zweiten Polyaminfraktion (N) verbleibende Anteil dieser Komponenten kann jedoch gemäß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewichte der Polyaminkomponenten von (A) zwischen der organischen Phase (B) beim Eintritt in den Extraktor (3) und der wäßrigen Phase (H) beim Verlassen des Extraktors (3).

Durch Zumischen von Anteilen der zweiten Polyaminfraktion (N), vorzugsweise als Teilstrom von (L) zum Ausgangsarylamin (A) kann dessen Gehalt an Komponenten von (G) relativ gesenkt und damit über das Verteilungsgleichgewicht die gemäß erster Variante erreichbare Untergrenze in diesen Komponenten im zweiten Teilprodukt (N) gesenkt werden. Die damit erzielte nur graduelle Verbesserung der Trennleistung mag für bestimmte Anwendungen ausreichend sein, geht in der Regel aber zu Lasten des Durchsatzes an (A).

Vorteilhafter und als Ausführungsform bevorzugt ist eine zweite Variante des erfindungsgemäßen Verfahrens, bei der sich zusätzlich auch in der zweiten Polyaminfraktion (N) die relative Anreicherung der in dieser Fraktion bevorzugt enthaltenen Komponenten weitgehend unabhängig von der ersten Produktfraktion, gezielt variieren läßt, indem die in der Extraktionsstufe (3) anfallende wäßrige Phase (H) oder zumindest eine Teilmenge derselben in einer nachgeschalteten, nach dem Gegenstromprinzip arbeitenden Extraktionsstufe (4) mit einer organischen Phase (O) extrahiert wird.

Die organische Phase (O) besteht im allgemeinen neben hydrophobem Lösungsmittel aus Hilfsamin und/ oder Polyamin, letzteres vorzugsweise mit der Zusammensetzung des zweiten Verfahrensteilproduktes (N).

Bei Verwendung einer organischen Phase (O) ohne Polyamin resultiert in der die Extraktionsstufe (4) verlassenden wäßrigen Phase (Q) eine Polyaminfraktion, in welcher die relative Anreicherung der in dieser Phase bevorzugt

enthaltenen Komponenten über die in der wäßrigen Phase (H) erzielte Anreicherung hinaus gezielt erhöht und maximiert werden kann, auf Kosten der Polyaminkonzentration in der wäßrigen Phase (Q).

Polyamin als Bestandteil der organischen Phase (O) bewirkt, daß die die Verfahrensstufe (4) verlassenden Phasen (P) und (Q) eine höhere und damit für die Durchführung des erfindungsgemäßen Verfahrens energetisch vorteilhaftere Polyaminkonzentration aufweisen, als bei Verwendung einer organischen Phase (O) ohne Polyamin.

Durch die bevorzugte Verwendung eines Polyamins mit der Zusammensetzung des zweiten Teilproduktes (N) als Bestandteil der organischen Phase (O) kann auch unter diesen energetisch vorteilhaften günstigen Bedingungen infolge Gleichgewichtseinstellung mit Selbstverstärkung des Trenneffektes die relative Anreicherung der in der die Trennstufe (4) verlassenden wäßrigen Phase (Q) bevorzugt enthaltenen Polyaminkomponenten und damit der zweiten Polyaminfraktion (N) variiert und maximiert werden.

Im einfachsten und allgemeinen Fall wird die organische Phase (O) gebildet aus zumindest einem Teilstrom von Mengenstrom (E) und gegebenenfalls weiterem Hilfsamin aus Mengenstrom (F). Vorzugsweise wird (O) gebildet aus einem Teilstrom von (E) und einer Teilmenge des Mengenstromes (L), enthaltend das Polyamingemisch (N) neben gegebenenfalls Hilfsamin und neben hydrophobem Lösungsmittel.

Gegebenenfalls kann vollständig auf die Zuspeisung von hydrophobem Lösungsmittel und Hilfsamin aus anderen Quellen verzichtet werden, so daß die organische Phase (O) ausschließlich aus einer Teilmenge von (L) besteht.

Diese Ausführungsform des erfindungsgemäßen Verfahrens ist bezüglich Trennleistung und Energiebilanz besonders vorteilhaft und daher besonders bevorzugt, wenn sie angewendet werden kann.

Die Molarität der in der nachgeschalteten Extraktionsstufe (4) eingesetzten wäßrigen Phase liegt im allgemeinen auf gleicher Höhe wie in der die Extraktionsstufe (3) verlassenden wäßrigen Phase (H). Es ist jedoch möglich durch Zugabe von Wasser gegebenenfalls auch von Hilfsamin zur wäßrigen Phase sowohl die Molarität als auch den Protonierungsgrad zur Verbesserung der verfahrensgemäßen Trennleistung der Extraktionsstufe (4) zu verändern.

Grundsätzlich ist es auch möglich, die Molarität der wäßrigen Phase in (4) durch Entzug von Wasser zu erhöhen.

Die in Stufe (4) resultierende organische Phase (P) wird der in Extraktionsstufe (3) eingesetzten organischen Phase (B) zugesetzt.

Die in Stufe (4) resultierende wäßrige Phase (Q) wird, gegebenenfalls mit dem Rest von (H) der Extraktionsstufe (5) zugeführt.

Für diejenige Menge an hydrophobem Lösungsmittel, welche gegebenenfalls zur Bildung des Extraktionsmittels (O) dem Lösungsmittelkreislauf des zweiten Verfahrensproduktes (N) durch Abzweigen eines Teilstromes von (L) entzogen wird, erfolgt ein Mengenausgleich vorzugsweise durch Zugabe einer Teilmenge von (E) bei der Bildung der organischen Phasen (J).

Mit der zweiten Variante des erfindungsgemäßen Verfahrens läßt sich die relative Anreicherung in beiden resultierenden Polyaminfraktionen gezielt variieren und maximieren. Neben dieser in qualitativer Hinsicht großen Vielseitigkeit und Leistungsfähigkeit bietet die zweite Verfahrensvariante zumindest für die zweite Polyaminfraktion (N) auch eine energetisch günstige Ausführungsform.

Der mit Gewinnung der ersten Polyaminfraktion (G) verbundene Aufwand steigt dagegen relativ um so stärker, je geringer der mengenmäßige Anteil von (G), bezogen auf eingesetztes Polyamingemisch (A) ist, weil der verbleibende Gehalt an Polyamin (G) in der destillativ aufzuarbeitenden organischen Phase (D) entsprechend immer kleiner wird.

Der Effekt kommt besonders dann zum tragen, wenn die mit (G) abgetrennten Komponenten im Ausgangsgemisch (A) nur in geringer Konzentration enthalten sind und/oder relativ hoch in der Fraktion (G) angereichert werden, z. B. bei der erfindungsgemäßen Auftrennung von Polyamingemischen der Diphenylmethanreihe.

Eine zusätzliche, teilweise Einbringung von Ausgangspolyamin (A) in der Trennstufe (3) über die wäßrige Phase bringt zwar in der Regel eine Erhöhung der Polyaminkonzentration in (D) und damit energetische Entlastung. Bei der erfindungsgemäßen Durchführung des Verfahrens ist diese Entlastung aber infolge der Gleichgewichtseinstellung zwischen dem Polyamin in der wäßrigen Phase und dem Polyamin in der organischen Phase (D) mit einer Verschlechterung des qualitativen Trennergebnisses im ersten Teilprodukt (G) verbunden, so daß von dieser Möglichkeit nur in untergeordnetem Maße oder in Fällen mit entsprechend geringen Anforderungen an das Trennergebnis Gebrauch gemacht wird.

Eine in dieser Hinsicht verbesserte Ausführung stellt die dritte Variante des erfindungsgemäßen Verfahrens dar. Ausgehend von der ersten Variante wird diese dahingehend erweitert, daß die die Verfahrensstufe (3) verlassende, das erste Teilprodukt (G) in gegenüber der Konzentration von (A) in (B) verringerter Konzentration enthaltende organische Phase (D) geteilt wird in einen Mengenstrom (D'), der weiterhin mit dem Ziel der Gewinnung der Polyaminfraktion (G) der Aufarbeitungsstufe (6) zugeführt wird, und in einen Mengenstrom (D").

Der Mengenstrom (D") wird in einer vorgelagerten Extraktionsstufe (2) mit zumindest einer Teilmenge, vorzugsweise mit der Gesamtmenge der zur Wiederverwendung zur Verfügung stehenden wäßrigen Phase (C) extrahiert.

Der dem Extraktor (2) zugeführte Mengenstrom (D") wird dabei so bemessen, daß bei der Umsetzung mit Mengenstrom (C) ein möglichst weitgehender, vorzugsweise praktisch quantitativer Übergang der in der organischen

Phase (D") enthaltenen Polyamine in die den Extraktor (2) verlassende wäßrige Phase erfolgt

Eine erhöhte Molarität in der in Stufe (2) eingesetzten wäßrigen Phase, resultierend aus Verfahrensstufe (8) des erfindungsgemäßen Verfahrens, begünstigt und erleichert den Übergang von Polyamin aus der organischen Phase (D") in die wäßrige Phase.

Der Restgehalt an Polyamin in der die Verfahrensstufe (2) verlassenden organischen Phase (R) liegt im allgemeinen bei < 5 Gew.-%, vorzugsweise bei < 1 Gew.-%.

Im übrigen richtet sich der in (R) zulässige Höchstgehalt an Amin und insbesondere der Gehalt an Polyamin nach den aus der jeweiligen Trennaufgabe resultierenden qualitativen Anforderungen an die Verfahrensprodukte, d. h. die Qualität der Trennung, im Falle der Variante 3 insbesondere an das Verfahrensteilprodukt (N). Die Einhaltung des für die Qualität von (N) relevanten Gehaltes an Polyamin wird im Rahmen der technischen Gegebenheiten unter Ausschöpfung der zur Verfügung stehenden wäßrigen Phase über die Bemessung des Teilmengenstromes (D") kontrolliert.

In quantitativer Hinsicht kommt es dem Verfahren und insbesondere der vorgelagerten Verfahrensstufe (2) zustatten, daß das Verhältnis von (D") zu (D') besonders dann zu höheren Werten tendiert, d. h. daß (D") mengenmäßig größer wird, wenn das Mengenverhältnis der Polyaminfraktion (G) zu (N) kleiner wird, weil (N) auf Kosten von (G) größer wird. Mit zunehmenden Anteil der zweiten Polyaminfraktion (N) erhöht sich die im Kreis geführte und damit in (2) zur Extraktion von (D") zur Verfügung stehende wäßrige Phase.

Die an Amin verarmte, insbesondere von Polyamin praktisch befreite, die Verfahrensstufe (2) verlassende organische Phase (R) wird der organischen Phase (B) zugeschlagen und zusammen mit dieser als Lösungsmittel für Ausgangspolyamin (A) der Verfahrensstufe (3) zugeführt.

Die die Verfahrensstufe (2) verlassende wäßrige Phase enthält neben der eingesetzten wäßrigen Säure Polyamin, welches in seiner Zusammensetzung weitgehend dem in (D) abgetrennten und als Fraktion (G) isolierten Polyamin entspricht und gegebenenfalls Hilfsamin.

Im einfachsten Falle wird die die Verfahrensstufe (2) verlassende wäßrige Phase direkt als Mengenstrom (C) der Verfahrensstufe (3) zugeführt; gegebenenfalls werden vorher weitere wäßrige Phase und/oder Wasser und/oder Hilfsamin zugemischt.

Auch die Zugabe einer begrenzten Menge von Ausgangspolyamin (A) zu der wäßrigen Phase ist gemäß Variante 3 möglich ohne Einbuße an qualitativer Trennleistung (relativer Anreicherung) gegenüber Variante 1, dafür aber bei verbesserter quantitativer Leistung (größerer Wirtschaftlichkeit).

Bei einer weiteren vierten Variante wird die vorgelagerte Verfahrensstufe (2) und die sich ergebenden Vorteile kombiniert mit der als Variante 2 beschriebenen Ausführungsform des erfindungsgemäßen Verfahrens, die dadurch bezüglich des ersten Teilproduktes eine analoge Verbesserung erfährt wie bei Variante 3 beschrieben. Als eine zusätzliche Erleichterung und Vereinfachung für die Durchführung der Verfahrensstufe (2) erweist es sich, daß die Anforderung an den verbleibenden Polyamingehalt der in (2) resultierenden organischen Phase (R) weniger streng sind, da die nachteiligen Auswirkungen eines erhöhten Polyamingehaltes in (R) auf die Qualität des zweiten Verfahrensteilproduktes (N) durch die nachgeschaltete Extraktionsstufe (4) kompensiert werden können.

Der Gehalt an Polyamin in der die Verfahrensstufe (2) verlassendenden organischen Phase (R) liegt daher im allgemeinen bei < 5 Gew.-%, vorzugsweise bei < 3 Gew.-%.

## Beispiel 1

Ausgangspolyamingemisch (A) (2,000 kg/h) wird mit dem im wesentlichen aus Polyamingemisch, Anilin und Xylol bestehenden Mengenstrom (B) (5,961 kg/h) vermischt.

Mengenstrom (B) wird gebildet aus Mengenstrom (P) (3,205 kg/h), Anteilen von Mengenstrom (E) und Anteilen von Mengenstrom (F).

Die resultierende organische Phase (Mengenströme A+B) hat im Mittel folgende Zusammensetzung:

| | |
|---|---|
| Mengenstrom (A)+(B)<br>( 7,961 kg/h) | 40,5 % Polyarylamin<br>15,8 % Anilin<br>42,6 % Xylol<br><0,1 % Chlorwasserstoff<br>1,1 % Wasser. |

und wird in einem mehrstufig wirkenden Extraktor (3) bei 90°C dem Mengenstrom (C) entgegengeführt, der die folgende durchschnittliche Zusammensetzung hat:

| | |
|---|---|
| Mengenstrom (C) ( 20,500 kg/h ) | 0,5 % Polyarylamin<br>15,8 % Anilin<br>3,7 % Chlorwasserstoff<br>80,0 % Wasser. |

Die den Extraktor (3) verlassende organische Phase (D) hat folgende durchschnittliche Zusammensetzung:

| | |
|---|---|
| Mengenstrom (D) ( 7,407 kg/h ) | 13,5 % Polyarylamin<br>38,3 % Anilin<br>45,8 % Xylol<br><0,1 % Chlorwasserstoff<br>2,4 % Wasser. |

Mengenstrom (D) wird im Extraktor (6.0) mit Wasser gewaschen. Das resultierende Waschwasser aus (6.0) [und analog (7.0) ] wird über den Mengenstrom (Y) an geeigneter Stelle mit der wässrigen Phase vereinigt.

Zur Sicherheit wird Mengenstrom (D) mit verdünnter Natronlauge gewaschen. Die wässrige Phase wird als Abwasser entsorgt.

Der gewaschene und von Säureresten befreite Mengenstrom (D) wird in einer ersten Destillationsstufe (6.1) vom größten Teil des Xylols und einem Teil des Anilins befreit. Das Destillat (Mengenstrom E) wird aufgeteilt und den Verfahrensstufen (3) und (5) zugeführt.

In der zweiten Destillationsstufe (6.2) wird aus der Sumpfphase von (6.1) das Anilin und restliches Xylol abdestilliert. Das Destillat wird aufgeteilt und den Verfahrensstufen (3) und (5) zugeführt.

Als Destillationssumpf von Stufe (6.2) verbleibt ein Polyamingemisch, welches als Mengenstrom (G) mit ca. 1,0 kg/h in Tank (9) gesammelt wird.

Die den Extraktor (3) verlassende wässrige Phase (H) hat folgende durchschnittliche Zusammensetzung:

| | |
|---|---|
| Mengenstrom (H) ( 21,054 kg/h ) | 11,1 % Polyarylamin<br>7,9 % Anilin<br>3,6 % Chlorwasserstoff<br>77,4 % Wasser. |

und wird in einem weiteren mehrstufig wirkenden Extraktor (4) bei 90°C einer organischen Phase (O) entgegengeführt, die eine Teilmenge der organischen Phase (L) ist.

Mengenstrom (O)
( 3,071 kg/h)

Die in Verfahrensstufe (4) resultierende organische Phase (P) geht ein in Mengenstrom (B).

| | |
|---|---|
| Mengenstrom (P) (3,205 kg/h) | 38,3 % Polyarylamin<br>19,5 % Anilin<br>39,7 % Xylol<br><0,1 % Chlorwasserstoff<br>2,4-% Wasser |

Die in (4) resultierende wässrige Phase (Q) wird in einem weiteren mehrstufig wirkenden Extraktor (5) bei 90°C dem Mengenstrom (J) entgegengeführt.

Mengenstrom (Q)
( 20,919 kg/h )

| | |
|---|---|
| Mengenstrom (J) (7,916 kg/h) | 54,4 % Anilin<br>45,1 % Xylol<br>0,5 % Wasser |

Mengenstrom (J) wird gebildet aus dem Destillatstrom (M) der Destillationsstufe (7.1) und entsprechenden Teilen der Destillatströme (E) und (F).

Die den Extraktor (5) verlassende organische Phase (L) fällt an mit der folgenden durchschnittlichen Zusammensetzung:

| | |
|---|---|
| Mengenstrom (L)<br>( 8,607 kg/h ) | 18,1 % Polyarylamin<br>37,9 % Anilin<br>41,5 % Xylol<br><0,1 % Chlorwasserstoff<br>2,5 % Wasser. |

Mengenstrom (L) wird aufgeteilt in Mengenstrom (O), welcher der Extraktionsstufe (4) zugeführt wird, und Mengenstrom (L').

Vor der destillativen Aufarbeitung in Verfahrensstufe (7.1) wird mit Mengenstrom (L') analog verfahren wie mit Mengenstrom (D).

Im anschließenden Destillationsschritt (7.1) werden Wasser, Xylol und Anilin von der als Destillationssumpf anfallenden zweiten Polyaminfraktion (N) abgetrennt.

Das in der Destillationsstufe (7.1) anfallende Destillat - gegebenenfalls nach mechanischer Abtrennung des beim Abkühlen des Destillates abgeschiedenen Wassers - wird zusammen mit den verbliebenen Teilströmen (E) und (F) zur Bildung des Extraktionsmittelstromes (J) verwendet und als solcher in der Extraktionsstufe eingesetzt.

Die Polyaminfraktion (N) wird als zweites Teilprodukt mit ca. 1,0 kg/h in Tank (10) gesammelt.

Der den Extraktor (5) verlassenden wässrigen Phase (K) wird anschließend in einem Wasserverdampfer (8) je nach Bedarf destillativ Wasser entzogen (Mengenstrom X), die zusammen mit dem aus den Destillatströmen von (6.1) und (7.1) gegebenenfalls abgetrennten Wasser zum Waschen der organischen Phasen in den Stufen (6.0) und (7.0) verwendet werden. Die resultierenden Waschwässer werden als Mengenstrom (Y) gesammelt und der in (8) resultierenden wässrigen Phase unter Bildung von Mengenstrom (C) zugesetzt.

Die über die Sicherheitsneutralisationsstufen bei der Wäsche mit Natronlauge dem System entzogenen Säure - und Wassermengen werden durch Zugabe zum Mengenstrom (C) von außen ersetzt.

| **Polyarylamin**<br>**GC:** | **A**<br>**[Gew.-%]** | **G**<br>**[Gew.-%]** | **N**<br>**[Gew.-%]** |
|---|---|---|---|
| 2,2'-Diamino-diphenylmethan | 0,22 | 0,44 | - |
| 2,4'-Diamino-diphenylmethan | 7,12 | 14,24 | <0,1 |
| 4,4'-Diamino-diphenylmethan | 60,20 | 20,90 | 99,50 |
| N-Methyl-4,4'-diamino-diphenylmethan | 0,21 | 0,42 | - |
| Σ-Diamino-diphenylmethane | 67,75 | 36,00 | 99,50 |
| Σ-Mehrkernpolyamine | 32,25 | 64,00 | 0,50 |
| Mengenverteilung | 100 % | 50 % | 50 % |

**Patentansprüche**

**1.** Verfahren zur Fraktionierung und Reinigung von aromatischen Polyamin-gemischen, verwendbar für die Herstellung von Polyisocyanaten **dadurch gekennzeichnet, daß** man

a) das Ausgangspolyamingemisch (A) in einem zweiphasigen System, bestehend aus (i) einer hydrophoben Lösungsmittelphase (B), die im wesentlichen aus hydrophobem Lösungsmittel und gegebenenfalls einem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und gegebenenfalls Polyaminen besteht, und (ii) einer wäßrigen Phase (C), bestehend im wesentlichen aus Wasser, einer starken Säure und zumindest teilweise in der Salzform vorliegendem Hilfsamin, sowie gegebenenfalls zumindest teilweise in der Salzform vorliegenden Polyaminen, unter Zuhilfenahme einer, nach dem Gegenstromprinzip arbeitenden Extraktionsstufe (3) unter Durchmischung der Phasen verteilt, indem man das Ausgangspolyamingemisch vorzugsweise über die organische Phase (B) in die Extraktionsstufe (3) einbringt, und die die Extraktionsstufe verlassende organische Phase (D), zumindest teilweise, gegebenenfalls nach Durchlaufen einer

Waschstufe (6.0) in einer mehrstufigen Destillation (6.1), (6.2) in eine erste, in der Extraktionsstufe (3) wiederverwendete Fraktion (E), bestehend im wesentlichen aus hydrophobem Lösungsmittel und gegebenenfalls Hilfsamin, eine zweite Fraktion (F), bestehend im wesentlichen aus Hilfsamin und gegebenenfalls hydrophobem Lösungsmittel, und einem Destillationsrückstand (G), bestehend im wesentlichen aus einer ersten Polyaminfraktion auftrennt und die die Extraktionsstufe (3) verlassende wäßrige Phase (H),

b) gegebenenfalls zumindest teilweise über eine zwischengeschaltete Extraktionsstufe (4)

c) in eine Extraktionsstufe (5) leitet, in welcher nach dem Prinzip der Gegenstromextraktion eine Extraktion der wäßrigen Phase mit einer aus Lösungsmittel und Hilfsamin bestehenden Lösungsmittelphase (J) erfolgt, und die an Polyarylamin verarmte wäßrige Phase (K) resultiert, die man

d) gegebenenfalls zumindest teilweise über eine zwischengeschaltete Destillationsstufe (8) führt und anschließend mit dem gegebenenfalls verbliebenen Rest von (K) vereinigt und

e) als Mengenstrom (C) wiederverwendet, in dem man besagte wäßrige Phase

f) gegebenenfalls zumindest teilweise über eine vorgeschaltete Extraktionsstufe (2)

g) der Extraktionsstufe (3) zuführt, gegebenenfalls nach Zugabe von Hilfsamin und/oder Wasser, und man

h) die in der Extraktionsstufe anfallende organische Phase (L) zumindest teilweise destillativ (7.1) in ein aus Hilfsamin und Lösungsmittel bestehendes Destillat (M) und einen aus einer zweiten Polyaminfraktion bestehenden Destillationsrückstand (N) zerlegt, wonach das Destillat (M) mit wenigstens einem Teil des in der Destillationsstufe (6.2) gewonnenen Destillates (F) vereinigt und anschließend zur Extraktionsstufe (5) zurückgeführt und dort wieder verwendet wird.

**2.** Verfahren gemäß Anspruch 1, **dadurch gekennzeichnet, daß** man
b) die in der Extraktionsstufe (3) anfallende wäßrige Phase (H) zumindest teilweise in einer zwischengeschalteten, nach dem Gegenstromprinzip arbeitenden Extraktionsstufe (4) extrahiert unter Verwendung einer organischen Phase (O) als Extraktionsmittel, bestehend neben hydrophobem Lösungsmittel aus Hilfsamin und gegebenenfalls Polyamin, letzteres vorzugsweise mit einer Zusammensetzung des zweiten Teilproduktes (N) und vorzugsweise eingebracht als Teilmenge des Mengenstromes (L), die in Verfahrensstufe (4) resultierende organische Phase (P) dem Mengenstrom (B) und damit der Extraktionsstufe (3) und die resultierende wäßrige Phase (Q) der Extraktionsstufe (5) zuführt.

**3.** Verfahren nach Anspruch 1 oder 2, **dadurch gekennzeichnet, daß** man
d) die die Extraktionsstufe (5) verlassende wäßrige Phase (K) vor ihrer Wiederverwendung zumindest teilweise destillativ (8) von einem Teil (X) des in ihr enthaltenen Wassers befreit, dieses gegebenenfalls zum Waschen (6.0) des der destillativen Aufarbeitung (6.1), (6.2) zugeführten Teiles der die Extraktionsstufe (3) verlassenden organischen Phase (D) und/oder zum Waschen (7.0) des der destillativen Aufarbeitung (7.1) zugeführten Teiles der die Extraktionsstufe (5) verlassenden organischen Phase (L) zwecks Entfernens von Säurespuren verwendet, und das hierbei anfallende Wasser (Y) an geeigneter Stelle in die wäßrige Phase zurückführt.

**4.** Verfahren gemäß einem oder mehreren der Ansprüche 1 bis 3 **dadurch gekennzeichnet, daß** man
f) eine Teilmenge (D") der die Extraktionsstufe (3) verlassenden organischen Phase (D) abtrennt und in einer vorgelagerten, vorzugsweise mehrstufigen, Extraktionsstufe (2) mit zumindest einer Teilmenge, vorzugsweise mit dem gesamten Mengenstrom (C) im Gegenstrom extrahiert, und den Teilmengenstrom (D") so bemißt, daß dabei ein möglichst weitgehender Übergang des in (D") enthaltenen Polyamins in die die Extraktionseinheit (2) verlassende wäßrige Phase stattfindet, besagte wäßrige Phase, gegebenenfalls nach Zugabe von Wasser aus Mengenstrom (Y) und/oder Hiflsamin und/oder weiterer wäßriger Phase aus Mengenstrom (C) der Extraktionsstufe (3) zuführt, und die in (2) anfallende organische Phase (R), bestehend im wesentlichen aus hydrophobem Lösungsmittel und gegebenenfalls Hilfsamin, ebenfalls der Extraktionsstufe (3) zuführt und als Bestandteil der organischen Phase (B) als Lösungsmittel für das Ausgangspolyamin (A) verwendet.

**5.** Verfahren gemäß einem oder mehreren der Ansprüche 1 bis 4 **dadurch gekennzeichnet, daß** man als Hilfsamin Anilin verwendet.

6. Verfahren gemäß einem oder mehreren der Ansprüche 1-5, **dadurch gekennzeichnet, dass** man als Polyamingemische ein Polyamingemische der Diphenylmethanreihe verwendet.

7. Verfahren gemäß einem oder mehreren der Ansprüche 1 bis 6 **dadurch gekennzeichnet, daß** man als Polyamingemisch der Diphenylmethanreihe ein Polyamingemisch verwendet, wie es bei der säurekatalysierten Anilin-/Formaldehyd-Kondensation anfällt.

8. Verwendung der gemäß den Ansprüchen 1 bis 7 behandelten aromatischen Polyamingemische zur Herstellung der entsprechenden aromatischen Polyisocyanatgemische.

9. Verwendung der gemäß den Ansprüchen 1 bis 7 behandelten aromatischen Polyamingemische zur Herstellung entsprechender kernhydrierter Polyamine oder als Vernetzer und Epoxidhärter.

10. Die Verwendung der gemäß den Ansprüchen 1 bis 7 behandelten aromatischen Polyamingemische zur Herstellung von Polyurethankunststoffen.

**Claims**

1. Process for the fractionation and purification of aromatic polyamine mixtures usable for the production of polyisocyanates, **characterised in that**

a) the polyamine starting mixture (A) is distributed in a two-phase system comprising (i) a hydrophobic solvent phase (B) which is composed substantially of hydrophobic solvent and optionally an aromatic auxiliary amine that is virtually insoluble in water and under normal pressure has a boiling point at least 20°C below the boiling point of the lowest boiling component of the starting mixture and at least 20°C above the boiling point of the solvent, and optionally of polyamines, and (ii) an aqueous phase (C) comprising substantially water, a strong acid and auxiliary amine present at least partially in the salt form, and optionally polyamines present at least partially in the salt form, with the assistance of an extraction stage (3) operating on the countercurrent principle with mixing of the phases, by introducing the starting polyamine mixture, preferably via the organic phase (B), into extraction stage (3), and at least partially separating the organic phase (D) leaving extraction stage, optionally after passing through a wash stage (6.0), in a multistage distillation (6.1), (6.2) into a first fraction (E) reused in extraction stage (3) and substantially composed of hydrophobic solvent and optionally auxiliary amine, a second fraction (F), substantially composed of auxiliary amine and optionally hydrophobic solvent and a distillation residue (G), substantially composed of a first polyamine fraction and the aqueous phase (H) leaving the extraction stage is passed,

b) optionally at least partially via an intermediate extraction stage (4),

c) into an extraction stage (5), in which the aqueous phase is extracted according to the countercurrent principle with a solvent phase (J) composed of solvent and auxiliary amine, and the aqueous phase (K) depleted in polyarylamine results, which is passed

d) optionally at least partially via an intermediate distillation stage (8) and then combined with the optionally remaining remainder of (K) and is reused

e) as stream (C), in which the stated aqueous phase,

f) optionally at least partially via an upstream extraction stage (2)

g) is introduced into extraction stage (3), optionally after the addition of auxiliary amine and/or water, and

h) the organic phase (L) obtained in the extraction stage is at least partially split by distillation (7.1) into a distillate (M) comprising auxiliary amine and solvent and a distillation residue (N) composed of a second polyamine fraction, whereupon the distillate (M) is combined with at least a proportion of the distillate (F) obtained in distillation stage (6.2) and is then returned to extraction stage (5) and there used again.

2. Process according to claim 1, **characterised in that**

b) the aqueous phase (H) arising in extraction stage (3) is at least partially extracted in an intermediate extraction stage (4) operating according to the countercurrent principle using an organic phase (O) as the extraction agent which is composed, in addition to hydrophobic solvent, of auxiliary amine and optionally polyamine, the latter preferably with a composition of the second partial product (N) and preferably introduced as a partial quantity of stream (L), the organic phase (P) arising in process stage (4) is introduced into the stream (B) and thus into extraction stage (3) and the resultant aqueous phase (Q) is introduced into extraction stage (5).

**3.** Process according to claim 1 or 2, **characterised in that**

d) before being re-used, the aqueous phase (K) leaving extraction stage (5) is freed at least partially from a proportion (X) of the water contained therein by distillation (8), this water being used optionally for washing (6.0) that proportion of the organic phase (D) leaving extraction stage (3) fed to working-up by distillation (6.1), (6.2), and/or for washing (7.0) that proportion of the organic phase (L) leaving extraction stage (5) fed to working-up by distillation (7.1) for the purpose of removing acid traces and the water (Y) obtained in so doing is returned to the aqueous phase at a suitable place.

**4.** Process according to one or more of claims 1 to 3, **characterised in that**

f) a partial quantity (D") of the organic phase (D) leaving extraction stage (3) is separated and extracted countercurrently in an upstream, preferably multistage, extraction stage (2) with at least a partial quantity, preferably with the entire stream of (C) and the partial stream (D") is calculated in such a manner that the polyamine contained in (D") is as far as possible transferred into the aqueous phase leaving the extraction unit (2), the said aqueous phase, optionally after the addition of water from stream (Y) and/or auxiliary amine and/or further aqueous phase from stream (C) is introduced into extraction stage (3) and the organic phase (R) arising in (2), which is substantially composed of hydrophobic solvent and optionally auxiliary amine, is also introduced into extraction stage (3) and, as a constituent of the organic phase (B), is used as a solvent for the starting polyamine (A).

**5.** Process according to one or more of claims 1 to 4, **characterised in that** the auxiliary amine used is aniline.

**6.** Process according to one or more of claims 1-5, **characterised in that** the polyamine mixtures used comprise a polyamine mixture of diphenylmethane series.

**7.** Process according to one or more of claims 1 to 6, **characterised in that** the polyamine mixture used is a polyamine mixture of the diphenylmethane series of the kind obtained during acid-catalysed aniline/ formaldehyde condensation.

**8.** Use of the aromatic polyamine mixtures treated according to claims 1 to 7 for the production of the corresponding aromatic polyisocyanate mixtures.

**9.** Use of the aromatic polyamine mixtures treated according to claims 1 to 7 for the production of the corresponding polyamines hydrogenated on the nucleus or as crosslinking agents and epoxy curing agents.

**10.** Use of the aromatic polyamine mixtures treated according to claims 1 to 7 for the production of polyurethane plastics.

## Revendications

**1.** Procédé de fractionnement et de purification de mélanges de polyamines aromatiques, utilisables pour la préparation de polyisocyanates, **caractérisé en ce que** :

a) on distribue en mélangeant les phases, le mélange de polyamines de départ (A) dans un système biphasique consistant en (i) un phase de solvant hydrophobe (B), qui consiste essentiellement en un solvant hydrophobe et, le cas échéant, une amine aromatique auxiliaire qui est pratiquement insoluble dans l'eau et présente sous pression normale, un point d'ébullition situé à au moins 20°C sous le point d'ébullition du composant à point d'ébullition le plus faible du mélange de départ et à au moins 20°C au-dessus du point d'ébullition du solvant, et le cas échéant des polyamines et (ii) une phase aqueuse (C), essentiellement constituée d'eau, d'un acide fort et d'amine auxiliaire présente au moins partiellement sous la forme de sel, ainsi que, le cas échéant, de polyamines présentes au moins partiellement sous forme de sel, en faisant appel à une étape d'extraction (3) travaillant selon le principe du contre-courant, en faisant passer le mélange de polyamines de départ de préférence par la phase organique (B) dans l'étape d'extraction (3) et en séparant au moins partiellement la phase organique (D) quittant l'étape d'extraction, le cas échéant après passage dans une étape de lavage (6.0), par une distillation de plusieurs stades (6.1), (6.2), en une première fraction (E), réutilisée dans l'étape d'extraction (3), essentiellement constituée de solvant hydrophobe et le cas échéant, d'amine auxiliaire, en une deuxième fraction (F), essentiellement constituée d'amine auxiliaire et le cas échéant, de solvant hydrophobe, et en un résidu de distillation (G), essentiellement constitué d'une première fraction de polyamine, et on conduit la phase aqueuse (H) quittant l'étape d'extraction (3),

b) au moins partiellement par une étape d'extraction intermédiaire (4)

c) vers une étape d'extraction (5), dans laquelle d'après le principe de l'extraction à contre-courant, il se déroule une extraction de la phase aqueuse avec une phase de solvant (J) consistant en solvant et amine auxiliaire, et il en résulte la phase aqueuse (K) appauvrie en polyarylamine, que

d) l'on conduit le cas échéant au moins partiellement vers une étape de distillation intermédiaire (8) et que l'on combine ensuite, avec le reste éventuel de (K), et

e) que l'on réutilise comme flux massique (C), dans lequel on amène la phase aqueuse considérée

f) facultativement en passant au moins partiellement par une étape d'extraction préalable (2)

g) dans l'étape d'extraction (3), le cas échéant après addition d'amine auxiliaire et/ou d'eau, et

h) on décompose la phase organique (L) produite dans l'étape d'extraction, au moins partiellement par distillation (7.1) en un distillat (M) constitué d'amine auxiliaire et de solvant et en un résidu de distillation (N) constitué d'une deuxième fraction de polyamine, après quoi le distillat (M) est combiné à au moins une partie du distillat (F) obtenu dans l'étape de distillation (6.2) et ensuite, ramené à l'étape d'extraction (5) et à nouveau utilisée dans celle-ci.

**2.** Procédé suivant la revendication 1, **caractérisé en ce que** :

b) on extrait la phase aqueuse (H) produite à l'étape d'extraction (3) au moins partiellement dans une étape d'extraction (4) intercalée, travaillant suivant le principe du contre-courant, en utilisant une phase organique (O) comme agent d'extraction, constituée en plus du solvant hydrophobe, d'amine auxiliaire et le cas échéant, de polyamine, cette dernière étant de préférence avec une composition du deuxième produit partiel (N) et de préférence, introduite en tant que partie du flux massique (L), on conduit la phase organique (P) résultant de l'étape (4) du procédé dans le flux massique (B) et ainsi, à l'étape d'extraction (3), et la phase aqueuse résultante (Q) dans l'étape d'extraction (5).

**3.** Procédé suivant la revendication 1 ou 2, **caractérisé en ce que** :

d) on libère la phase aqueuse (K) quittant l'étape d'extraction (5), avant sa réutilisation, d'une partie (X) de l'eau qu'elle contient, au moins partiellement par distillation (8), on utilise cette eau pour séparer les traces d'acide, le cas échéant pour le lavage (6.0) de la partie amenée au traitement par distillation (6.1), (6.2), de la phase organique (D) quittant l'étape d'extraction (3), et/ou pour le lavage (7.0) de la partie amenée au traitement par distillation (7.1) de la phase organique (L) quittant l'étape d'extraction (5), et on ramène l'eau (Y) produite à cette occasion à un endroit approprié dans la phase aqueuse.

**4.** Procédé suivant l'une ou plusieurs des revendications 1 à 3, **caractérisé en ce que** :

f) on sépare une partie (D'') de la phase organique (D) quittant l'étape d'extraction (3) et dans une étape d'extraction (2) préalable, de préférence en plusieurs étapes, on extrait à contre-courant avec au moins une partie, de préférence la totalité du flux massique (C) et on mesure le courant partiel (D'') de sorte qu'un transfert le plus étendu possible de la polyamine contenue dans (D'') se produise vers la phase aqueuse quittant l'unité d'extraction (2), on conduit la phase aqueuse considérée, le cas échéant après addition d'eau du flux massique (Y) et/ou d'amine auxiliaire et/ou d'une autre phase aqueuse du flux massique (C) à l'étape d'extraction (3) et on conduit également à l'étape d'extraction (3) la phase organique (R) produite en (2), essentiellement constituée de solvant hydrophobe et le cas échéant, d'amine auxiliaire, et on l'utilise en tant que constituant de la phase organique (B) comme solvant de la polyamine de départ (A).

**5.** Procédé suivant l'une ou plusieurs des revendications 1 à 4, **caractérisé en ce que** l'on utilise l'aniline comme amine auxiliaire.

**6.** Procédé suivant l'une ou plusieurs des revendications 1 à 5, **caractérisé en ce que** l'on utilise comme mélange de polyamines, un mélange de polyamines de la série des diphénylméthanes.

**7.** Procédé suivant l'une ou plusieurs des revendications 1 à 6, **caractérisé en ce que** l'on utilise comme mélange de polyamines de la série des diphénylméthanes, un mélange de polyamines tel que celui qui est produit dans la

condensation aniline/formaldéhyde sous catalyse acide.

8. Utilisation des mélanges de polyamines aromatiques traités suivant les revendications 1 à 7, pour la préparation des mélanges de polyisocyanates aromatiques correspondants.

9. Utilisation des mélanges de polyamines aromatiques traités suivant les revendications 1 à 7, pour la préparation des polyamines hydrogénées sur les cycles correspondantes ou comme agent de réticulation et durcisseur d'époxyde.

10. Utilisation des mélanges de polyamines aromatiques traités suivant les revendications 1 à 7, pour la préparation de matières plastiques en polyuréthanne.

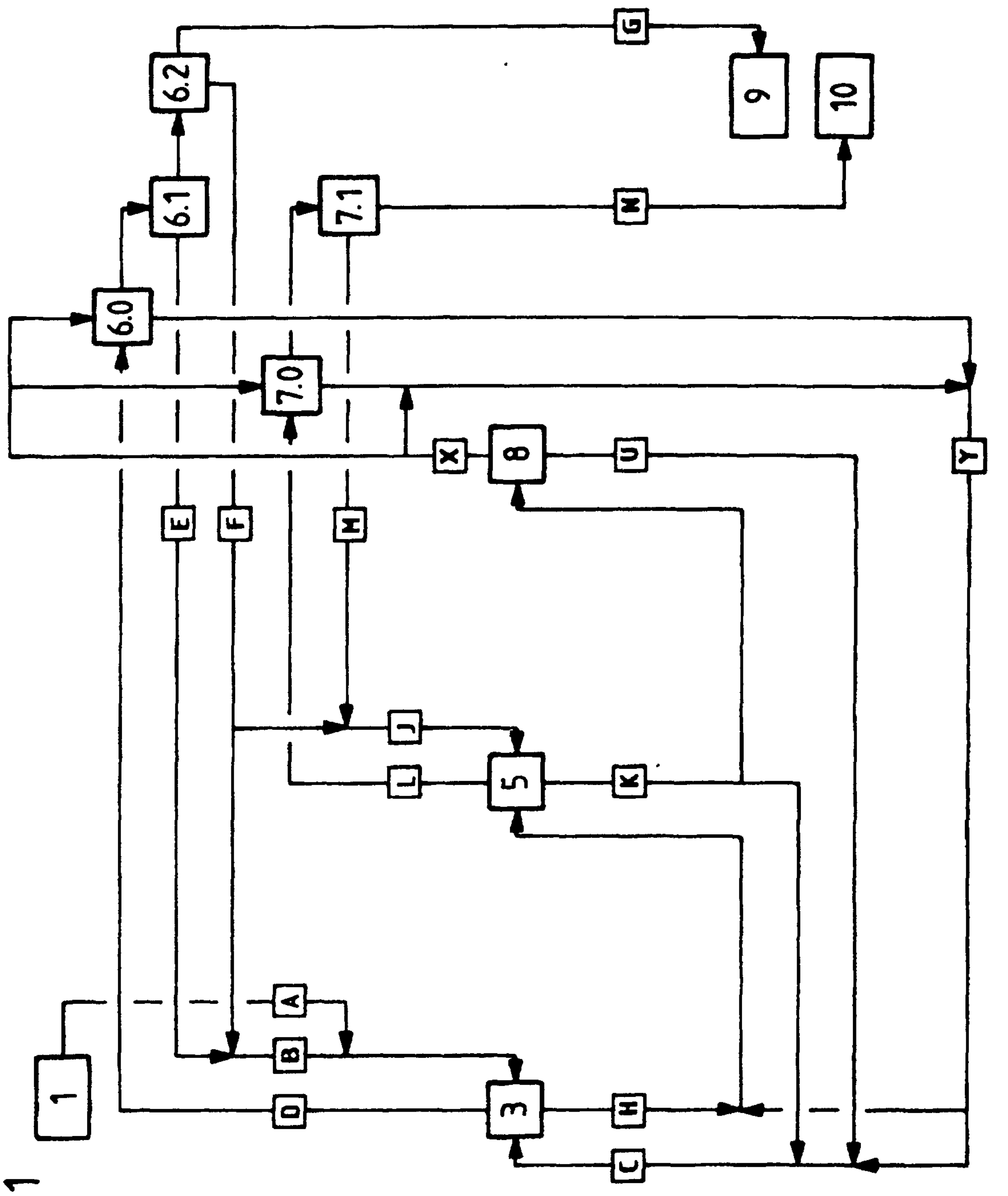


Fig.1

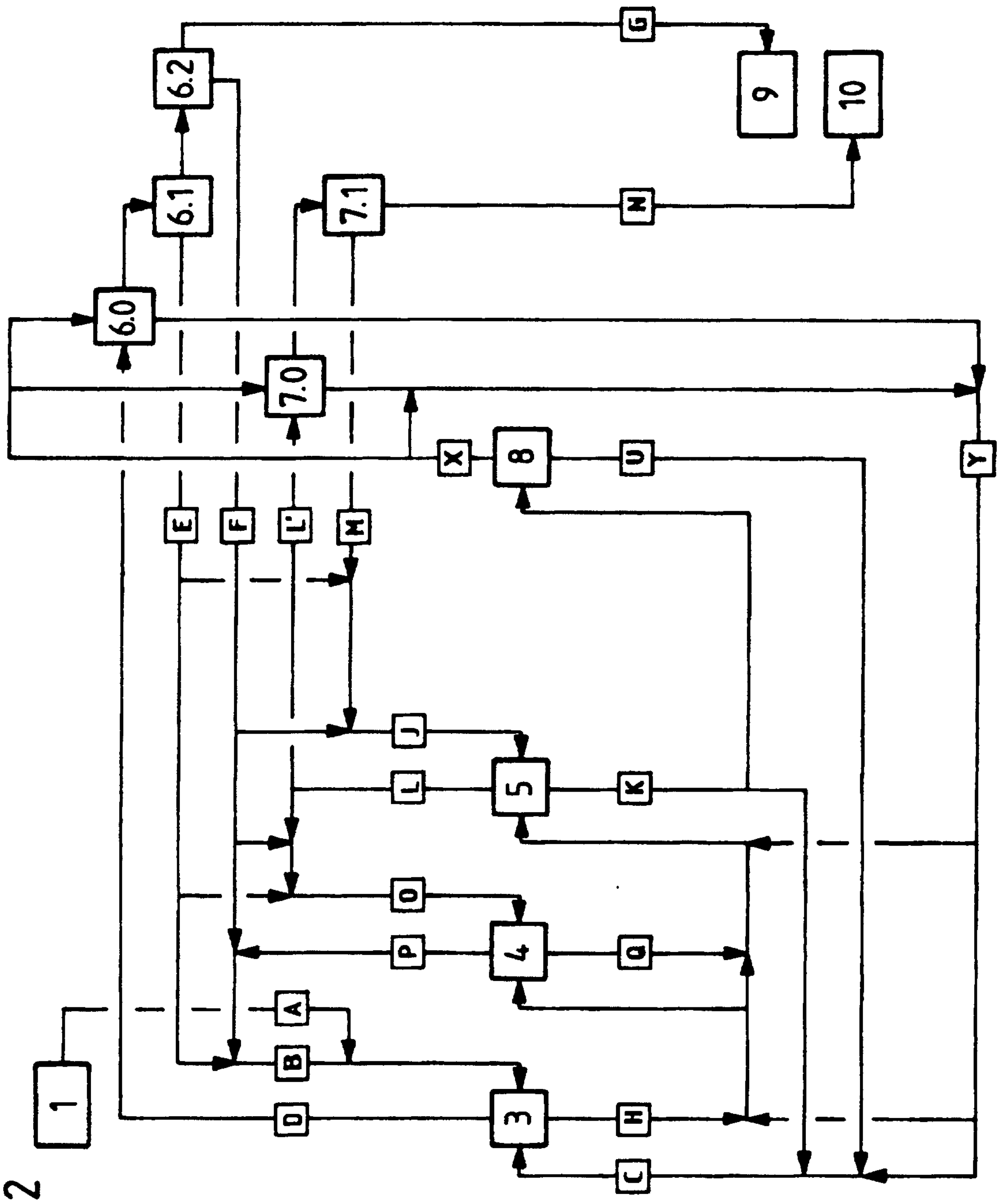


Fig.2

Fig. 3

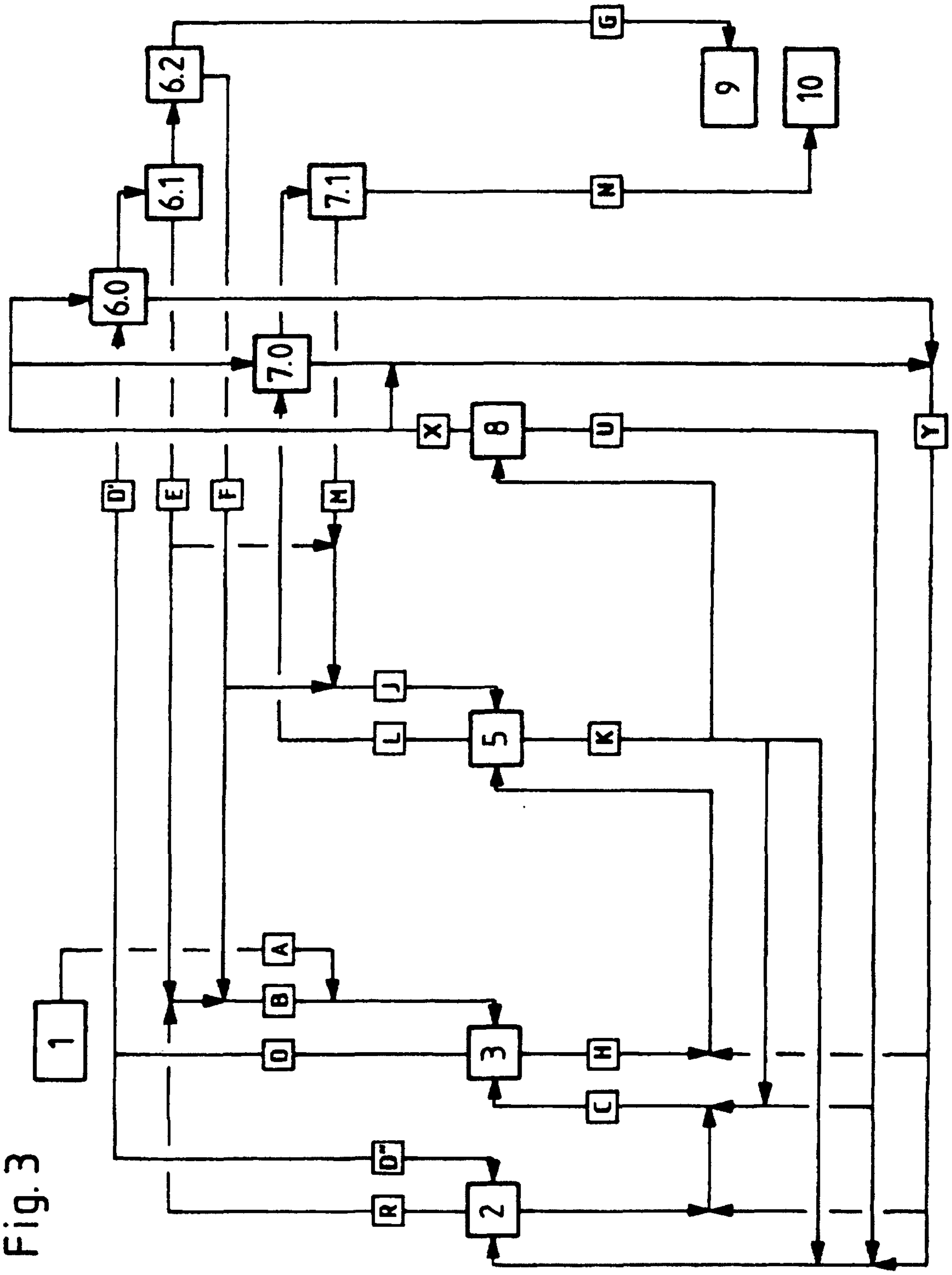

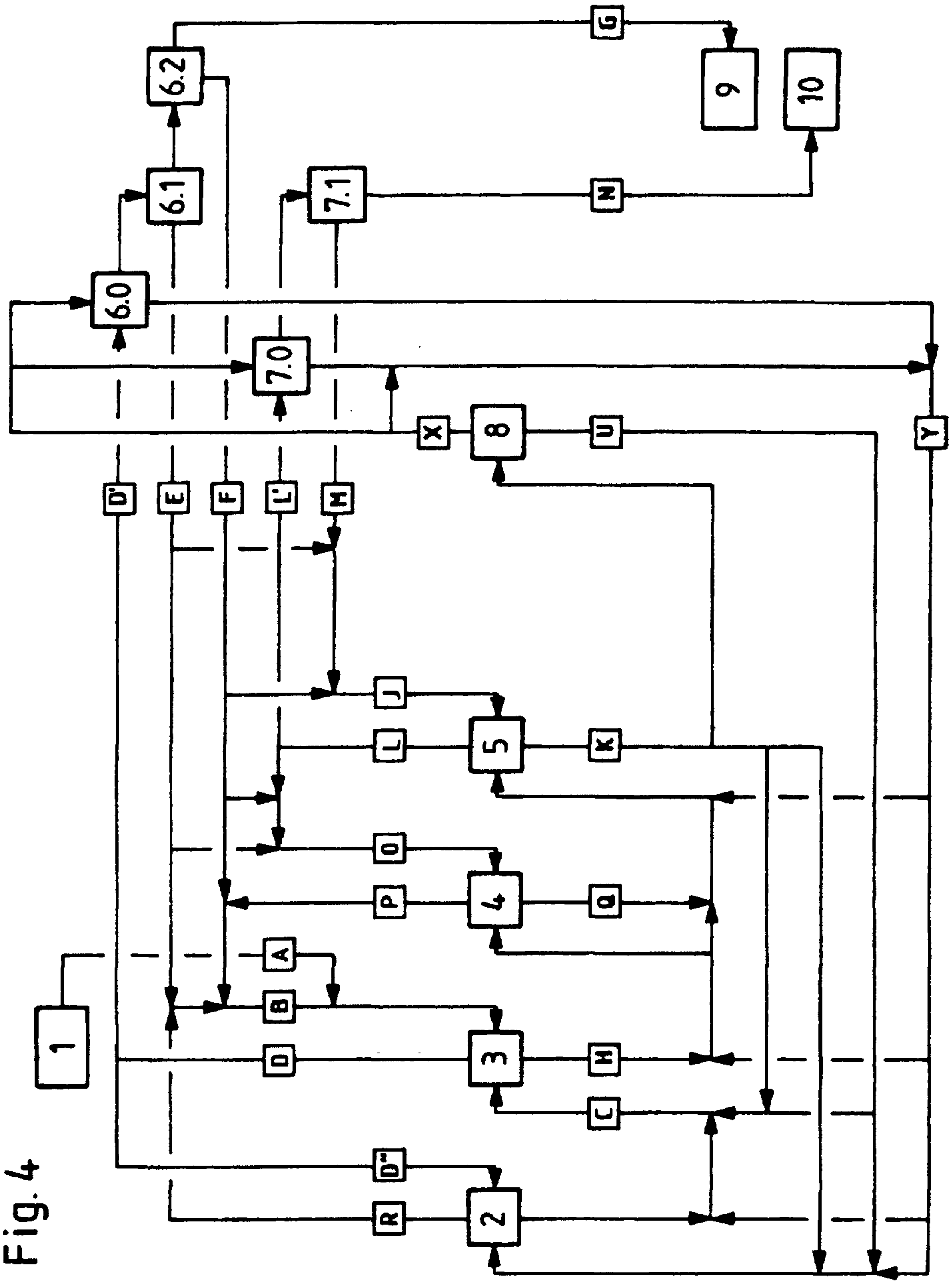


Fig. 4